# EUROPÄISCHE PATENTSCHRIFT

(11) **EP 1 140 257 B1**
(45) Veröffentlichungstag und Bekanntmachung des Hinweises auf die Patenterteilung: **24.07.2002**
(21) Anmeldenummer: 99962231.9
(22) Anmeldetag: 07.12.1999
(51) Int. Cl.: A61M 5/142, F04B 43/12, F04B 43/00

(54) **PUMPSCHLAUCHSYSTEM ZUR PERISTALTISCHEN FÖRDERUNG VON FLÜSSIGEN UND GASFÖRMIGEN MEDIEN**
PERISTALTIC PUMP TUBE SYSTEM FOR PUMPING GASEOUS AND LIQUID MEDIA
SYSTEME DE TUYAUX DE POMPE POUR L'ACHEMINEMENT PERISTALTIQUE DE MILIEUX LIQUIDES ET GAZEUX

(30) Priorität: 09.12.1998 DE 19856744
(43) Veröffentlichungstag der Anmeldung: 10.10.2001
(73) Patentinhaber: Rheotec AG, 9403 Goldach (CH)
(72) Erfinder: SCHIMMELPFENNIG, Winfried, D-18292 Krakow am See (DE); RIGGERS, Wilfried, D-27432 Bremervörde (DE)
(74) Vertreter: Blum, Hans-Werner
(86) Internationale Anmeldenummer: EP9909572
(87) Internationale Veröffentlichungsnummer: WO0033898

(56) Entgegenhaltungen:
- GB-A- 2 273 533
- US-A- 3 774 762
- US-A- 3 999 891
- US-A- 4 382 753

## Beschreibung

Die Erfindung betrifft ein Pumpschlauchsystem zur peristaltischen Förderung von flüssigen und gasförmigen Medien durch Rollen- bzw. Peristaltikpumpen.

In vielen medizinischen Geräten und Geräten der Labordiagnostik werden neben MembranPumpen (z.B. EP 0024431, W092/12345) vorwiegend Rollenpumpen (US 54434451) und Peristaltikpumpen (z.B. DE19529894) für verschiedene Förderaufgaben, vorwiegend zum Transport von Flüssigkeiten eingesetzt. Beide befördern das Medium, indem ein dafür vorgesehenes Pumpschlauchsegment von außen partiell dicht gequetscht wird und diese Quetschstelle dann in Förderrichtung bewegt wird, so daß sich das Fördergut im Schlauchsegment ebenfalls weiterbewegen muß. Bevor diese Dichtstelle den Schlauch am Ende des Pumpsegmentes wieder freigibt, wird am Anfang des Pumpsegmentes die nächste Dichtstelle geschaffen, die sich dann mit der nächsten Förderportion weiterbewegt.
Der Vorteil dieses Verfahrens liegt darin, daß keine Pumpenteile das Fördergut berühren, da sie keinen direkten Kontakt zum gepumpten Medium haben. Das Schlauchsystem mit den Pumpsegmenten ist meist ein steriles Einwegprodukt, das nach der Benutzung, z.B. nach einer Patientenbehandlung, entsorgt wird. Eine Krankheitsübertragung von einem Patienten zum nächsten ist so sicher vermeidbar.
Besonders in komplexen medizinischen Geräten enthält ein Schlauchsystem oft viele Pumpsegmente für verschiedene Pumpaufgaben. Eine Verwechslung eines Pumpsegmentes mit einem anderen hätte für den Patienten katastrophale Folgen und muß beim Einlegen des Schlauchsystems sicher vermieden werden, ebenso eine Vertauschung der Einlege- und damit der Pumprichtungen. Besonders in medizintechnischen Geräten, die einen extrakorporalen Blutkreislauf des Patienten beinhalten, wie Dialyse- und Blutplasma-Behandlungs-Systeme, ist diese Forderung oft lebenswichtig für den angeschlossenen Patienten. Deshalb sind bei vielen Anwendungen die Pumpsegmente mit aufwendigen mechanischen Paßstücken und Codierungen versehen, die ein fehlerhaftes Einlegen des umfängreichen Schlauchsystems verhindern (Beispiel FRESENIUS Dialysegerät A2008 mit Zusatzaufbau CMS08).

Die Anzahl der im Schlauchsystem vorhandenen Pumpsegmente richtet sich nach der Aufgabenstellung des Gerätes, es sind in der Medizintechnik Systeme mit weit mehr als zehn Pumpen und Schlauchklemmventilen im Einsatz (Beispiel Plasmaseparationseinheit BAXTER TPS mit Plasmabehandlungsgerät THERASORB PTI), in der Labordiagnostik werden bis zu 40 Kanäle gleichzeitig verwendet (Beispiel Labor-Kassettenpumpen). Die Komplexität dieser Schlauchsysteme führt häufig zu einem schwer überschaubaren Gewirr an Schläuchen, Pumpsegmenten und Verbindungselementen. Das richtige Einlegen aller Pumpsegmente ist zeitraubend und wird insbesondere hier zum Problem.
Die aufwendige Herstellung eines solchen Schlauchsystems ergibt sich durch die große Anzahl an Schlauchstücken und Verbindungselementen, die meist einzeln geklebt werden. Jede Klebestelle kann hierbei zu einem Dichtigkeitsproblem führen. Außerdem sind Toträume und Sackgassen im Schlauchsystem durch die räumlich entfernte Anordnung von Pumpen oft nicht zu vermeiden.
Deshalb werden in modernen Systemen (THERASORB PTI) die Pumpen und auch die Klemmventile, die den Fluß im Schlauchsystem steuern, in sogenannten Schlauchkassetten zusammengefaßt. Hier werden einzelne Schlauchsegmente des Schlauchsystems in stabilen Rahmen nebeneinander angeordnet (eingerastet) und mit dem Rahmen zusammen (z.B. US 5460493A) auf speziellen Pump- oder Magnet-Klemmeinheiten plaziert (z.B. US 5443451A). Diese Kassettenmethode ermöglicht ein verwechslungssicheres und schnelleres Einlegen, ändert jedoch nichts an der Kompliziertheit und den Risiken der Herstellung des Schlauchsystems aus einer Unzahl von Schlauchstücken und Verbindungsmuffen.

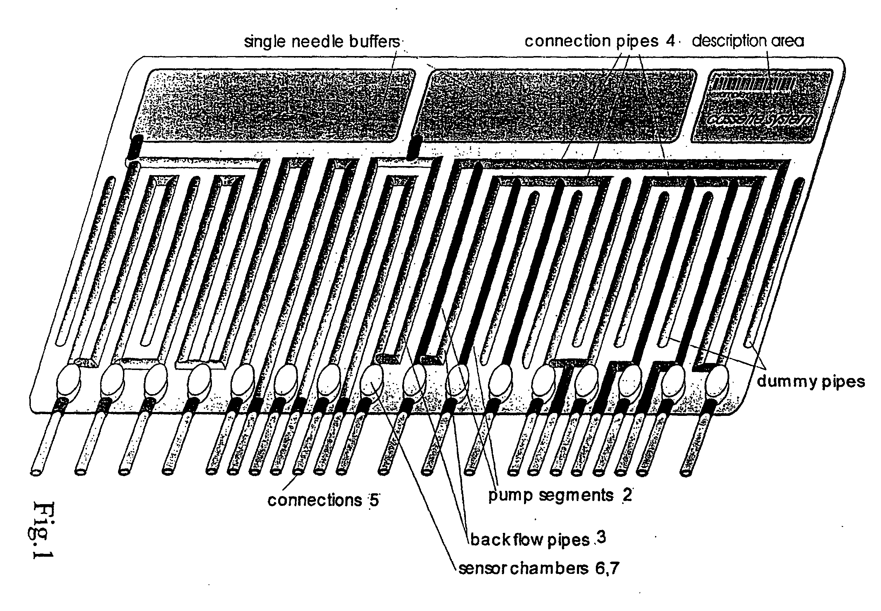

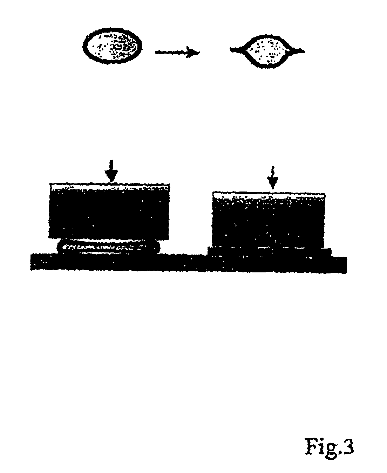

Im US-Patent US-A-3774762 wird vorgeschlagen, komplette Schlauchsysteme aus Folienmaterial zu prägen (Fig. 1), wobei alle Kammern, Verbindungen und Sensoren Bestandteile dieser Folienstruktur sind. Zum Bewegen der verschiedenen Flüssigkeiten werden Kammern mechanisch leergedrückt oder eine Walzenkette mit Rollen drückt den Inhalt eines Foliensegmentes weiter (Fig. 3). Der Querschnitt dieser Struktur weist kreisförmig geprägte übliche Schlauchstrukturen auf, die durch Ventilstößel oder durch die Rollen der Walzenkette von außen geschlossen werden. Obwohl die US-Patentschrift den Gedanken der Schlauchkassette aus Folienmaterial mit einer geprägten Flußplanstruktur offenbart, konnte die beschriebene Vorrichtung auf Grund ihrer Kompliziertheit nicht zur Anwendung kommen. Darüber hinaus ist sie für Rollen- bzw. Peristaltikpumpen ohnehin nicht anwendbar. Schließlich muß darauf hingewiesen werden, daß der Verschluß des Folienmaterials hier nur mit großem Kraftaufwand möglich ist. Seitliche Verschiebungen des Folienmaterials durch das Zusammenpressen der geprägten Folienkanäle sollen durch eingearbeitete Fixierstifte verhindert werden (Fig. 3).

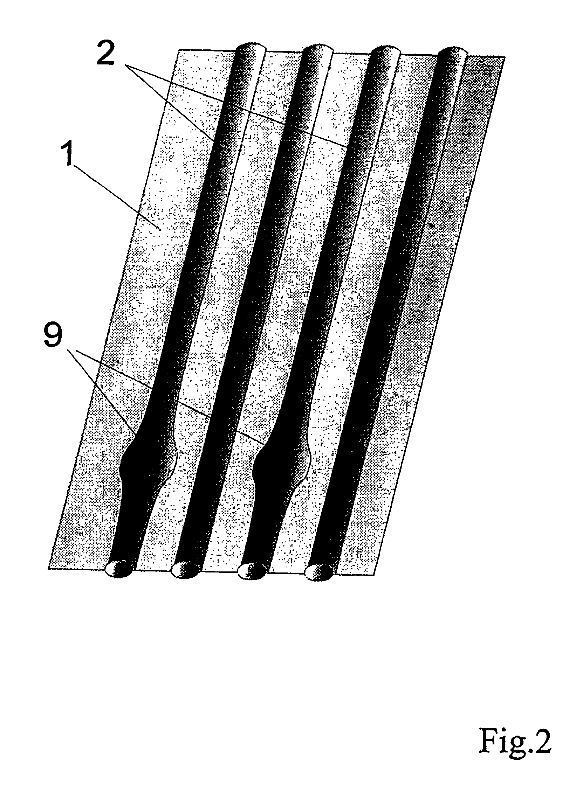

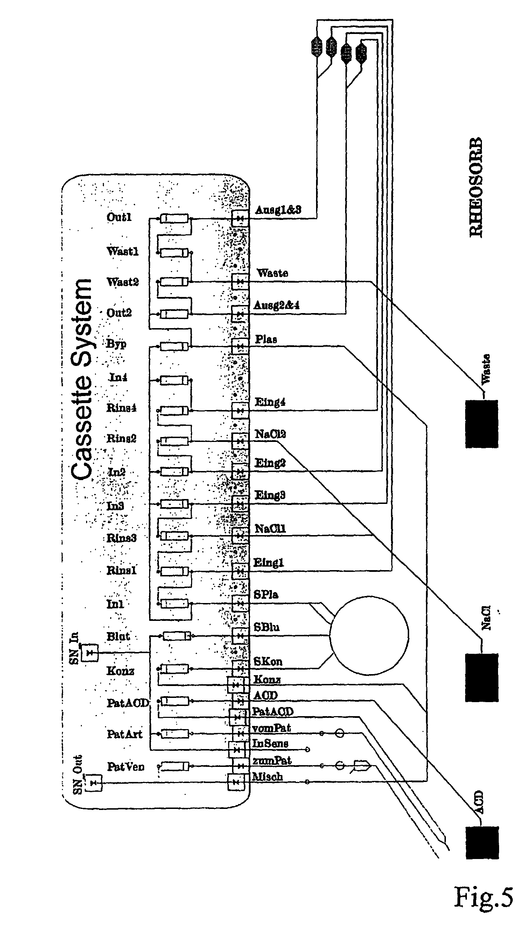

Ein weiteres generelles Problem von Schlauchpumpen ist die beschränkte Lebensdauer der Pumpsegmente. Dadurch, daß der im Querschnitt eigentlich runde Schlauch zyklisch plattgewalzt wird, besteht die Gefahr, daß er speziell an seinen seitlich extrem belasteten Biegestellen vorzeitig bricht und deshalb undicht wird. Die Pumprollen müssen ja, um Druck im Schlauch aufbauen zu können, den Pumpschlauch so zudrücken, daß die Druckstelle wirklich dicht geschlossen ist, eine Okklusion auf Abstand Null. Durch diese Dichtigkeitsforderung im Pumpsegment ist der Biegeradius seitlich am Pumpsegmentquerschnitt beim Quetschen sehr klein, ein echter Knick. Daher sind generell nur wenige sehr flexible Materialien für diese Anforderungen als Pumpsegment geeignet (z.B. Silikon). Problematisch ist oft der druckdichte Anschluß dieser Materialien an das übrige Schlauchmaterial (meist Weich-PVC). Schläuche werden für Pumpaufgaben vorwiegend deshalb eingesetzt, weil sie als Meterware ökonomisch produziert werden können. Im US-Patent 4382753 wird eine Pumpe beschrieben, bei der das Pumpsegment aus zwei verschweißten Folien geprägt ist. Das Grundprinzip entspricht einer Membranpumpe, eine flexible Kammer saugt durch ein Eingangsventil Flüssigkeit an, dann wird dies geschlossen, das Ausgangsventil geöffnet und die Kammer leergedrückt. Durch eine nachgeschaltete zweite Kammer mit kleinerem Volumen wird gleichzeitig eine Gegenbewegung erzeugt, die das zyklische Pumpen ausgangsseitig weitgehend linearisiert. Besonders interessant ist hierbei die Querschnittsform des Verbindungskanals (Fig. 5), da hier von der Kreisform abgewichen wird. Durch das Prägen der Folien ist für die Ventile eine für den Verschluß günstigere Form entstanden, eine Ellypse mit zweit seitlichen Ecken. Dadurch ist die Lebensdauer dieser Quetschventile höher als bei Schlauchmaterial und die Schließkraft ist geringer, da die Ecken schon vorgeknifft sind. Diese Form des Ventils wird auch im GB-A-2273533 benutzt (Fig. 2), hier wird das Pumpsegment jedoch um zwei weitere Eingangsventile erweitert, es kann so wahlweise aus einer der drei verschiedenen Eingangsflüssigkeiten gepumpt werden.

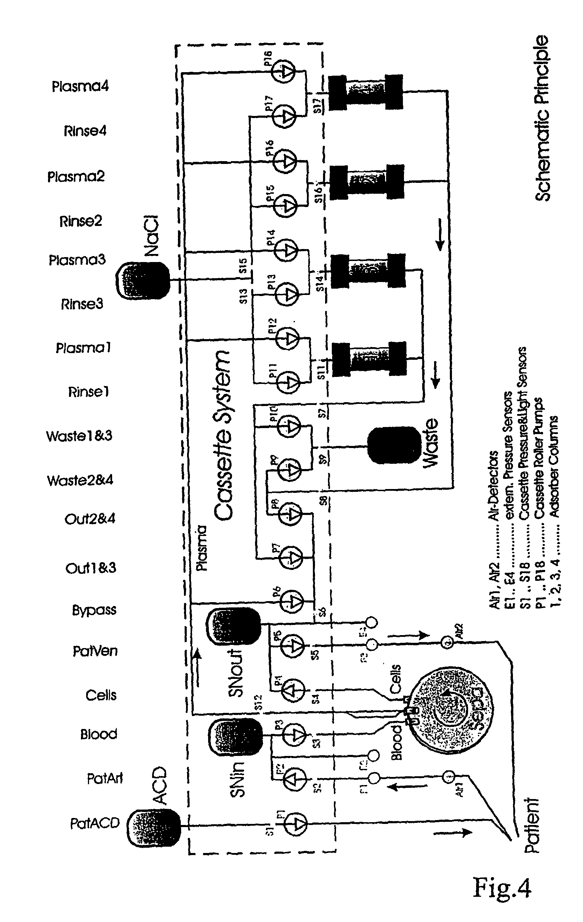

Ein weiteres generelles Problem von Schlauchpumpen resultiert aus dem Umstand, daß jede zur Förderung notwendige Quetschung des Pumpsegmentes durch eine Rolle am Ende des Pumpsegmentes wieder aufgehoben wird, kurz nachdem am Anfang des Pumpsegmentes die nächste Quetschstelle dicht geschlossen ist (Druck-Übergabe an die nächste Rolle).
Dieses Freigeben des Schlauches am Pumpsegment-Ende führt zu einem kurzzeitigen Rückströmen des bereits geförderten Materials, da das Schlauchvolumen sich dort wieder füllen muß, wo die Rolle das Pumpsegment verläßt, der gequetschte Schlauch wird dort wieder rund. Daher haben einfache Rollen- und Peristaltikpumpen eine systembedingte Diskontinuität im Fluß, sie fördern nicht gleichmäßig. Dies stellt ein wichtiges Problem für viele potentielle Anwendungsfälle dar, in denen ein konstanter pulsationsfreier Fluß vorausgesetzt werden muß. Mit erhöhtem Aufwand, z.B. zwei phasenverschoben fördernden Pumpsegmenten, deren Pulsationen sich annähernd aufheben (DE 3726452) oder speziellen Einlauf- bzw. Auslaufformen des verlängerten Pumpengegenlagers (DE 4135609) wird versucht, eine Fluß-Linearisierung zu erreichen.
Im US-Patent US-A-3999891 wird über eine komplizierte Gegen-Drehbewegungsmechanik (Fig. 1) der zusätzlich hydraulisch angepreßten Andruckrollen erreicht, daß der Fluß bei jeder Umdrehung des Pumprades in mehrere einzelne Portionen zerlegt und so annähernd linearisiert wird. Die Pumpsegmente sind generell Schläuche mit kreisförmigem Querschnitt (Fig. 1, Fig. 5, Fig. 6). Fig. 4 zeigt einen Drucksensorbalg, der zur Pumpensteuerung über einen Anschlußschlauch an den Ausgang der Pumpe anzuschließen ist. Der mechanische Aufwand für diese Linearisierungsmethode erscheint sehr hoch.

Der Erfindung liegt daher die Aufgabe zugrunde, ein Pumpschlauchsystem zur Anwendung bei Rollen- bzw. Peristaltikpumpen zu beschreiben, das die beschriebenen Mängel überwindet.
Dies betrifft die Verwechslungssicherheit von Pumpen durch Anwendung des Kassettenprinzips bei nebeneinander angeordneten Pumpschlauch- und Rollenpumpsegmenten, die wesentlich vereinfachte und kostengünstige Herstellbarkeit des Schlauchsystems, die prinzipiell größere Lebensdauer der Pumpschlauchsegmente bei gleicher Materialwahl sowie die Linearisierung der Flußcharakteristik der einzelnen Pumpschlauchsegmente der Kassette.

Erfindungsgemäß wird diese Aufgabe durch die Merkmale des Anspruches 1 gelöst. Vorteilhafte Ausgestaltungen ergeben sich aus den weiteren Ansprüchen.

Die Schlauchkassette besteht aus einer Doppelfolie, die über ein spezielles Herstellungsverfahren gegensätzlich geprägt und miteinander verschweißt ist (s. Fig.1).
Eine solche Schlauchkassette beinhaltet keine einzelnen Schlauchstücken mehr, sondern die geformten internen Kanäle übernehmen viele Funktionen des Gesamtschlauchsystems. So sind in ihr alle Pumpschlauchsegmente integriert, weiterhin alle vom Gesamtflußschema definierten Verbindungen der Segmente untereinander. Sogar Sensorkammem für Druck- und optische Transmisionssensoren sind ausprägbar sowie Kammern zur Zwischenspeicherung von Pumpmedien (z.B. Single-Needle-Volumina bei extrakorporalen Blutanwendungen). Hierdurch entfallen fast alle an üblichen Schlauchsystemen nötigen Verbindungsmuffen, Y- und T-Stücke und deren Verklebungen. Gleichzeitig wird durch die räumliche Nähe aller Pumpschlauchsegmente das Füllvolumen des Gesamtschlauchsystems minimal, was z.B. bei extrakorporalen Detoxikationsbehandlungen an Kindern (z.B. Dialyse, Intensivmedizin) sehr wichtig ist.
Auch systembedingte Toträume und Sackgassen im Schlauchsystem sind so, in direkter Nachbarschaft aller Pumpen, minimierbar.
Ähnlich, wie eine Leiterplatte, die in der Elektronik systematisch herkömmliche Verbindungsdrähte ersetzt, so ersetzt die Schlauchkassette ein herkömmliches umfangreiches Schlauchsystem.
Dadurch, daß zwischen zwei benachbarten Pumpschlauchsegmenten immer noch ein weiteres Schlauchsegment ausgeprägt wird (z.B. zur Rückleitung der hinübergepumpten Flüssigkeit), wird erreicht, daß alle Anschlüsse der Kassette an der gleichen Seite liegen können. Gleichzeitig dienen diese Zwischensegmente dazu, während des Pumpbetriebs die seitlichen Materialverschiebungen durch die Quetschung der benachbarten Pumpsegmente als ovale Verformungen des Segmentquerschnittes aufzufangen. Deshalb wird, wenn nötig, ein zusätzliches Dummy-Segment ohne weitere Funktion zwischen zwei Pumpschlauchsegmenten eingefügt (s. Fig.1).
Der Kostenaufwand zur Herstellung einer solchen Folienkassette liegt weit unter den Fertigungskosten für ein herkömmliches Schlauchsystem mit gleicher Komplexität.

Dadurch, daß keine Schläuche mehr als Pumpschlauchsegmente verwendet werden, ist nun ohne zusätzlichen Aufwand im Prägewerkzeug eine bessere Formgebung des Pumpensegmentes möglich.

Um die Lebensdauer der Segmente zu erhöhen, wurde ihr Querschnitt so ausgebildet, daß sich die Form eines leicht offenen Mundes ergibt, einschließlich der spitzen Mundwinkel (s.Fig.3). Diese Form ist für eine Okklusion durch äußere Kräfte viel besser geeignet, als die übliche Kreisform eines Schlauches, da sie keine Knickstelle mehr aufweist. Alle Biegeradien des Querschnittes sind so bemessen, daß das Schlauchmaterial beim Pumpen nur in elastischer Hinsicht verformt wird. Die zur Okklusion des Pumpsegmentes nötigen Kräfte sind wesentlich geringer, als bei einem runden Schlauch gleicher Wandstärke, auch dadurch ist die Lebensdauer der Folien-Pumpsegmente bei gleicher Pumpleistung entscheidend höher.

Zur Flußlinearisierung wurde ebenfalls die einfache Formbarkeit der Pumpschlauchsegmente bei der Herstellung im Prägewerkzeug genutzt.
Bei jeder Rollenpumpe gibt es den Moment, wo die fördernde Rolle vom Pumpschlauchsegment abhebt und die nächste Rolle die Förderung der nächsten Portion beginnt. Durch das Abheben der vorderen Rolle wird im Pumpsegment jedoch Volumen angefordert, die Gesamtförderung würde verringert. Dies wird jedoch ausgeglichen, indem die nachfolgende Rolle am Eingang des Pumpensegmentes genau in diesem Moment über eine definiert geprägte Querschnittserweiterung (Verdickung 9) rollt (Fig.2), also pro Winkeleinheit genau so viel Volumen zusätzlich fördert, wie die vordere Rolle beim Abheben gerade anfordert. Dadurch bleibt die Gesamtförderung am Pumpsegment-Ausgang linear.

Im folgenden sind Ausführungsbeispiele der Erfindung dargestellt. Sie wird anhand eines komplexen Schlauchsystems zur extrakorporalen Blut-Plasmaseparations- und Adsorptionsbehandlung näher erläutert. In der zugehörigen Zeichnung zeigen:
- Fig. 1: Doppelfolien-Schlauchkassette mit eingeprägten Pumpschlauchsegmenten, Verbindungen und Kammern
- Fig. 2: Formgebungen der Pumpschlauchsegmente zur Flußlinearisierung
- Fig. 3: Formgebung der Pumpsegment-Querschnitte
- Fig. 4: Flußschema eines Kassettensystems für die Aufgabenstellung Extrakorporales Blutplasma-Separations- und -reinigungsverfahren mit vier Adsorbern (RHEOSORB),
- Fig. 5: Kreuzungsfreies Flußschema (Entflechtung) für das Kassettensystem gemäß Fig. 4.

In Fig. 1 ist eine aus einer Doppelfolie geprägte Schlauchkassette 1 mit eingeprägten Pumpschlauchsegmenten 2 dargestellt. Zur Zu- und Rückführung des Transportgutes zur gegenüberliegenden Kassettenseite sind zwischen den Pumpschlauchsegmenten 2 Schlauchsegmente 3 in die Schlauchkassette 1 eingeprägt. Im Randbereich der Schlauchkassette befinden sich Verbindungen 4 der Pumpschlauch- und Schlauchsegmente 2, 3 sowie Anschlüsse 5 für die Verbindung mit externen Schlauchleitungen. Ausgeformte Druckkammern 6 in den geprägten Leitungen der Schlauchkassette 1 dienen zur Messung des internen Druckes im Transportgut durch externe Kraftsensoren. Die Schlauchkassette 1 weist ferner ausgeformte Kammern 7 zur optischen, akustischen oder anderweitigen Transmissionsmessung des Transportgutes auf.

Zur Linearisierung der Flußcharakteristik im Inflow- und/oder Outflow-Bereich der Pumpen besitzen die Pumpsegmente die in Fig. 2 dargestellte Form. Sie gewährleistet, daß beim Verlassen des Schlauchsegmentes der einen Andruckrolle einer Rollenpumpeneinheit durch die nächste Andruckrolle genau der Volumenbetrag zusätzlich gefördert wird, wie die vordere Rolle gerade durch das Abheben freigibt.

In Fig. 3 ist auf der linken Seite die bisher übliche Querschnittsform der Pumpschlauchsegmente, auf der rechten Seite die mit der Erfindung vorgeschlagene Querschnittsform dargestellt. Die neue Querschnittsform ist so ausgestaltet, daß die obere und untere Prägefolie seitlich in einem spitzen Winkel aufeinandertreffen und damit das Pumpschlauchsegment so vorgeformt ist, daß beim Abrollen der Andruckrollen das Plattdrücken an keiner Stelle des Umfanges zu einem Knicken des Folienmaterials führt.

Fig. 4 zeigt das Flußbild, insgesamt wird für die Therapie eine Kassettenpumpe mit 18 Pumpkanälen eingesetzt. Die Schlauchkassette übemimmt in diesem komplexen Schlauchsystem alle Pumpsegmente, alle Verbindungen der Pumpen untereinander, 18 Druckmeßkammern, sowie zwei Vorratskammern zur Single-Needle-Behandlung. Fig.5 zeigt die kreuzungsfreie Ausgestaltung des Schlauchsystems für das Prägewerkzeug.
Die Prozeduren zur Ermittlung des Folienkassetten-Layouts aus dem Flußbild sind die gleichen, wie sie zur Entwicklung einer einlagigen Leiterplatte aus einem Stromlaufplan benutzt werden (einlagige Entflechtung).
Zum Therapiebeginn wird diese Schlauchkassette auf die Pumpeinheit aufgelegt, der Deckel, der als Pumpengegenlager wirkt, wird über der Kassette geschlossen, alle Pumpen befinden sich nun im direkten Eingriff zu ihren Pumpschlauchsegmenten. Verwechslungen sind so ausgeschlossen, die Patientensicherheit ist gewährleistet, die Therapie kann sofort beginnen.
Dadurch, daß die Schlauchkassette alle notwendigen Querverbindungen des Schlauchsystems enthält, sind nur noch direkte Anschluß-Schläuche zu den verschiedenen anderen Baugruppen des Schlauchsystems an die Kassette angeschlossen, z.B. die Anschlüsse zum Patienten, zur Plasmaseparationseinheit, zu den Adsorbern und zu den Spülflüssigkeiten.
Neben diesen Pump- und Verbindungsfunktionen gibt es in der Kassette an jedem Pumpsegment eine ausgeprägte Druckkammer, die über externe Sensoren die Messung des Innendrukkes ermöglicht. Auch die für eine sogenannte Single-Needle-Behandlung notwendigen Vorratsvolumina wurden in der Schlauchkassette durch eingeprägte Kammern realisiert.

Die Kassette ist so gestaltet, daß sich nur minimale Toträume und Sackgassen im Schlauchsystem befinden, so daß die Spülung und der Wechsel von Fördermedien mit minimalen Rest-Durchmischungen gewährleistet ist. Die Verluste (Blut- bzw. Plasmaverluste) bei jedem Spülzyklus werden so ebenfalls minimal. Die Sicherheit des Kassettensystems wird durch Druckhaltetests während der Therapie zyklisch überwacht und gewährleistet.

## Patentansprüche

1. Pumpschlauchsystem zur peristaltischen Förderung von flüssigen und gasförmigen Medien, bestehend aus einer durch thermoplastische Formgebung hergestellten Schlauchkassette (1) mit eingeprägten Schlauch- und Kammersegmenten (2, 3, 6, 7) und ihren Verbindungen (4), **gekennzeichnet durch** eine Schlauchkassette (1) für die Förderung des flüssigen oder gasförmigen Mediums **durch** nebeneinander angeordnete Rollen- bzw. Peristaltikpumpen, die eine Vielzahl von parellelen, nach dem Flußbild des Pumpschlauchsystems eingeprägten Pumpschlauchsegmenten (2) aufweist, von denen jedes Pumpschlauchsegment einer Pumpe zugeordnet ist und daß zwischen zwei benachbarten Pumpschlauchsegmenten (2) immer ein weiteres Schlauchsegment (3) angeordnet ist, das der Leitung des Transportgutes und/oder dem Auffangen der seitlichen Verschiebungen der Pumpschlauchsegmente (2) dient.

2. Pumpschlauchsystem nach Anspruch 1, **dadurch gekennzeichnet, daß** alle Anschlüsse der Schlauchsegmente (2, 3) auf einer Seite der Schlauchkassette (1) angeordnet sind.

3. Pumpschlauchsystem nach Anspruch 1, **dadurch gekennzeichnet, daß** die Pumpschlauchsegmente (2) zur Linearisierung der Flußcharakteristik eine solche Formgebung und Querschnittsvergrößerung (9) aufweisen, daß beim Verlassen des Schlauchsegmentes einer der Andruckrolle einer Rollenpumpe durch die nächste Andruckrolle genau der Volumenbetrag zusätzlich gefördert wird, wie die vordere Rolle gerade durch das Abheben freigibt.

4. Pumpschlauchsystem nach Anspruch 1, **dadurch gekennzeichnet, daß** die geprägten Schlauchsegmente (2, 3) eine Querschnittsform aufweisen, die so ausgestaltet ist, daß die obere und untere Prägefolie seitlich in einem spitzen Winkel aufeinandertreffen und alle Biegeradien des Querschnitts so bemessen sind, daß das Schlauchmaterial beim Pumpen nur in elastischer Hinsicht verformt wird und beim Abrollen der Andruckrollen das Plattdrücken an keiner Stelle des Umfanges zu einem Knicken des Folienmaterials führt.

5. Pumpschlauchsystem nach Anspruch 1, **dadurch gekennzeichnet, daß** in die geprägten Schlauchsegmente (2, 3) bzw. in die eingeprägten Verbindungen (4) der Schlauchsegmente (2, 3) Druckkammern (6) zur Messung des internen Drucks im Transportgut integriert sind.

6. Pumpschlauchsystem nach Anspruch 1, **dadurch gekennzeichnet, daß** in die geprägten Schlauchsegmente (2, 3) bzw. in die eingeprägten Verbindungen (4) der Schlauchsegmente (2, 3) Kammern (7) zur optischen, akustischen und anderweitigen Transmissionsmessung des Transportgutes integriert sind.

## Claims

1. A pump tube system for the peristaltic delivery of liquid and gaseous media, consisting of a tube cassette (1), produced by a thermoplastic moulding process, with impressed tube and chamber segments (2, 3, 6, 7) and their connections (4), **characterised by** a tube cassette (1) for the delivery of the liquid and gaseous medium by roller or peristaltic pumps, arranged side by side, which has a large number of parallel pump tube segments (2) impressed in accordance with the flow pattern of the pump tube system, of which each pump hose segment is assigned to a pump and such that between two adjacent pump tube segments (2) there is always arranged a further pump tube segment (3) which serves to convey the product being transported and/or to intercept the lateral movements of the pump tube segment (2).

2. Pump tube system in accordance with Claim 1, **characterised by** all connections for the tube segments (2, 3) being arranged on one side of the tube cassette (1).

3. Pump tube system in accordance with Claim 1, **characterised by** the pump tube segments (2) having a shape and increase in cross-sectional area (9) for the linearisation of the flow characteristics, such that as one compression roller of a roller pump leaves the tube segment, the volumetric amount additionally delivered by the next compression roller is precisely that just released by the preceding roller lifting off.

4. Pump tube system in accordance with Claim 1, **characterised by** the impressed pump segments (2,3) having a cross section shape which is designed such that the upper and lower embossed foil meet at the sides at an acute angle and all bend radii of the section are dimensioned such that during pumping the tube material is only deformed from an elastic point of view and that when the compression rollers roll off this does not lead to kinking of the foil material at any point on the circumference.

5. Pump tube system in accordance with Claim 1, **characterised by** pressure chambers (6) for measuring the internal pressure in the transported product being integrated in the impressed tube segments (2,3) or in the impressed connections (4) of the tube segments (2, 3).

6. Pump tube system in accordance with Claim 1, **characterised by** chambers (7) for the transmission measurement of the transported product by visual, acoustic and other methods being integrated in the impressed tube segments (2,3) or in the impressed connections (4) for the tube segments (2, 3).

## Revendications

1. Système flexible de pompage destiné au refoulement péristaltique de fluides liquides et gazeux, composé d'une cassette flexible (1) fabriquée par moulage thermoplastique et présentant des segments tubulaires et des segments à chambres empreints (2, 3, 6, 7) avec leurs raccordements (4), **caractérisé par** une cassette flexible (1) pour le refoulement du fluide liquide ou gazeux par des pompes à rouleaux ou pompes péristaltiques juxtaposées et qui présente de nombreux segments tubulaires de pompage (2) parallèles empreints selon le diagramme d'écoulement du système flexible de pompage dont chaque segment flexible est attribué à une pompe, et qu'un autre segment flexible (3) servant à conduire et/ou à saisir les décalages latéraux des segments de segments tubulaires de pompage (2) est toujours disposé entre deux segments tubulaires de pompage (2) contigus.

2. Système flexible de pompage selon la revendication 1, **caractérisé par** en ce que tous les raccordements des segments tubulaires (2, 3) sont disposés sur un côté de la cassette flexible (1).

3. Système flexible de pompage selon la revendication 1, **caractérisé par** en ce que les segments tubulaires de pompage (2) destinés à la linéarisation de la caractéristique d'écoulement présentent un façonnage et une augmentation de la section transversale (9) tels que l'un des galets presseurs d'une pompe à rouleaux, en quittant le segment flexible, fait débiter en outre autant de volume par le prochain galet presseur que le galet presseur précédent libère en se soulevant.

4. Système flexible de pompage selon la revendication 1, **caractérisé par** en ce que les segments tubulaires (2, 3) empreints présentent une forme de section conçue de telle sorte que les feuilles à empreindre supérieure et inférieure se rencontrent latéralement en formant un angle aigu et que tous les rayons de courbure de la section transversale soient mesurés de telle sorte que le matériau flexible ne soit déformé au pompage que du point de vue de son élasticité et qu'au déroulement des galets presseurs, l'aplatissement ne conduise à un flambage du matériau constituant la feuille à un aucun endroit de la circonférence

5. Système flexible de pompage selon la revendication 1, **caractérisé par** en ce que des chambres sous pression (6) destinées à mesurer la pression interne du produit sont intégrées dans les segments tubulaires (2, 3) ou dans les raccordements empreints (4) des segments tubulaires (2, 3).

6. Système flexible de pompage selon la revendication 1, **caractérisé par** en ce que des chambres (7) destinées à mesurer la transmission optique, acoustique et autre du produit sont intégrées dans les segments tubulaires (2, 3) ou dans les raccordements empreints (4) des segments tubulaires (2, 3).
